# EUROPÄISCHE PATENTANMELDUNG

(11) **EP 0 858 811 A2**
(43) Veröffentlichungstag der Anmeldung: **19.08.1998**
(21) Anmeldenummer: 97123046.1
(22) Anmeldetag: 31.12.1997
(51) Int. Cl.: A61L 29/00, A61L 31/00, A61L 27/00

(54) **Gegenstand mit Mikroorganismen abweisender Beschichtung, dessen Herstellung und Verwendung**

(30) Priorität: 14.02.1997 DE 19705579
(71) Anmelder: HÜLS AKTIENGESELLSCHAFT, 45764 Marl (DE)
(72) Erfinder: Anders, Christine, Dr., 45721 Haltern (DE)

(57) **Zusammenfassung**

Die Erfindung betrifft einen Gegenstand aus Metall, Keramik oder Kunststoff, dessen Verwendung eine von Mikroorganismen möglichst freie Oberfläche erfordert und der gekennzeichnet ist durch eine Mikroorganismen abweisende Beschichtung aus cyanethylierter Hydroxyalkylzellulose. Die Beschichtung kann durch Plasma-Behandlung, Corona-Entladung oder Bestrahlung mit energiereichen Strahlen modifiziert werden. Der Gegenstand eignet sich zur Verwendung für medizinische oder technische Zwecke, beispielsweise als Katheter oder Stent.

## Beschreibung

Die Erfindung betrifft einen Gegenstand aus Metall, Keramik oder Kunststoff, dessen bestimmungsgemäße Verwendung eine von Mikroorganismen möglichst freie Oberfläche erfordert, sowie die Herstellung und die Verwendung eines solchen Gegenstands.

Oberflächen spielen bei biologischen Reaktionen auf zellulärer Ebene eine wichtige Rolle. So ist die Fähigkeit von Bakterien, auf der Oberfläche von Kunststoffen, Keramik und Metallen zu siedeln, ein ernstes Problem in der modernen Medizin. Besonders die häufig angewandten Katheter sind bevorzugte Siedlungsplätze für Bakterien (z.B. DA Goldmann und GB Pier, Clin.Microbiol.Rev. **6**, 176-192, 1993). Neben Kathetern werden im Körper von Patienten auch andere Gegenstände zeitlich begrenzt oder dauerhaft implantiert, wie Drainagen, Stents, Blutgefäßersatz, Zahnersatz, Herzklappen, Gelenkersatz, Kontaktlinsen, künstliche Linsen und chirurgisches Nahtmaterial. Diese Gegenstände bestehen aus Kunststoffen, Metall oder Keramik. Bei einer Besiedelung mit Bakterien müssen sie wieder aus dem Körper entfernt werden, weil sich oral oder intravenös verabreichte Antibiotika häufig als ungeeignet erwiesen haben, diese Bakterien zu bekämpfen. Ursache dafür dürfte die vielfach gesteigerte Resistenz der adhärierenden Bakterien gegen Antibiotika sein. (AG Gristina et al., Biomaterials, **8**, 423-426, 1987). Es wurde schon auf verschiedene Weise versucht, Kunststoffkatheter gegen Besiedelung durch Bakterien auszurüsten. Dabei werden z.B. Silbernitrat, Silberzeolith und Silbersulfadiazin eingesetzt (EP 328 421, JA 03244663, J. Infectious Diseases **167**, 920-924, 1992)

Auch die Inkorporation oder Belegung der Oberfläche mit Antibiotika mit hohen Konzentrationen wurde versucht in der Hoffnung, so lokal ausreichende Konzentrationen an Antibiotika zu erreichen, die höher sind als die MIC-Werte (= minmal inhibitory concentration) der adhärierenden Bakterien. Besonders Gentamycin, Rifampicin und Ciprofloxacin wurden dazu herangezogen (JM Schierholz, Hospitalis **65**, 403-407, 1995). Auch Antiseptika, wie Biguanindine **(?)**, Jod und/oder Chlorhexidine, wurden auf der Kunststoffoberfläche fixiert (G Golomb u A Shpigelman, J.Biomed.Mat.Res., **25**, 937-952, 1991). Dabei müssen jedoch lokale Konzentrationen von 0`1 bis 2% erreicht werden, um eine abtötende Wirkung zu erzielen. Bei diesen Konzentrationen werden aber auch körpereigene Zellen, wie Fibroblasten, irreversibel geschädigt. Außerdem ist es kaum möglich derart hohe Konzentrationen über einen längeren Zeitraum aufrechtzuerhalten. Insgesamt muß man feststellen, daß es bisher weder befriedigend gelungen ist, die Besiedelung der Oberflächen von Instrumenten und Hilfsmitteln für medizinische Zwecke mit Mikroorganismen zu verhindern oder zumindest wirksam zurückzudrängen, noch war es bisher möglich, eine einmal erfolgte Besiedelung in vivo wirksam und nachhaltig zu bekämpfen.

Bei der Herstellung von Arzneimitteln sowie in Forschungs- und Untersuchungslaboratorien. die mit biologischen Materialien arbeiten, können sich ebenfalls auf der Oberfläche von Apparaten, Behältern, Rohrleitungen, Flaschen, Pipetten usw., die in Kontakt mit sterilen wäßrigen Medien stehen, Bakterien ansiedeln und vermehren. Wenn es hier auch auch weniger problematisch ist, diesen schädlichen Befall zu beseitigen, so wäre es natürlich besser, wenn Materialien zur Verfügung ständen, die eine Besiedelung der Oberflächen gar nicht erst zulassen oder zumindest erheblich erschweren.

Ein weiteres Problem in der Medizin stellen Zellen oder zellenähnliche Partikel im Blut dar, z.B. Thrombozyten, die wie Bakterien eine starke Affinität zu Oberflächen aus Kunststoffen, Keramik oder Metall haben. Die Adhäsion von Thrombozyten auf der Oberfläche von Gegenständen aus den genannten Materialien, die als zeitweilige oder permanente Implantate oder sonstige Vorrichtungen oder Hilfsmittel in ständigem Kontakt mit Blut stehen, löst die Reaktionskaskade aus, die zu einer Verklumpung des Blutes und zur Thrombusbildung führt. Diese Adhäsion kann durch Antibiotika nicht beeinflußt werden. Zwar sind bestimmte Desinfektionsmittel, wie Benzalkoniumchlorid, in Verbindung mit Heparin in der Lage, die Adhäsion von Thrombozyten auf Kunststoffoberflächen zu reduzieren. Die Beschichtung aus Benzalkoniumchlorid/Heparin kann jedoch nur adsorptiv aufgebracht werden und löst sich relativ leicht ab. Es wäre daher erwünscht, eine unlösliche Beschichtung für die Oberfläche der genannten Materialien zu finden, die nicht nur die Adhäsion von Bakterien, sondern auch die von Zellen oder zellähnlichen Blutpartikeln, wie Thrombocyten, unterbindet.

Eine weitere wichtige Eigenschaft von Kunststoffen in der Medizintechnik, insbesondere bei Verwendung für intravasale Katheter, z.B. für Dilatationskatheter für verengte Herzkranzgefäße, ist die einer weichen Oberfläche mit geringem Reibungskoeffizienten. Die innere Zellauskleidung von Blutgefäßen, die sogenannte Endothelzellschicht, darf durch den eingeführten Katheter nicht beschädigt werden, da sich durch den Kontakt mit den darunterliegenden kollagenhaltigen Gewebeschichten leicht ein Thrombus ausbildet.

Weiterhin sind Bakterienansiedelungen in Wasseraufbereitungsanlagen (z.B. zur Entsalzung durch Membranen) oder auch in Behältern bekannt, die mit gelösten oder flüssigen unverdünnten organischen Substanzen gefüllt sind und für Bakterienpopulationen vorteilhafte Bedingungen aufweisen. Solche mikrobiellen Belegungen können in erheblichem Umfang zur Blockierung und/oder korrosiven Zerstörung der Anlage führen.

Besondere Bedeutung kommt dem Schutz vor Bakterienanhaftung und -ausbreitung in der Ernährung, der Pflege, hier insbesondere in der Altenpflege, und in der Medizin zu. Bei Massenbeköstigungen oder -ausschank existieren besonders dann erhebliche Risiken, wenn zur Vermeidung von Abfall von Einweggeschirr abgesehen wird und eine nur unzureichende Reinigung des Mehrweggeschirrs erfolgt. Die schädliche Ausbreitung von Bakterien in lebensmittelführenden Schläuchen und Rohren ist ebenso bekannt wie die Vermehrung in Lagerbehältern sowie in Textilien in feuchter und warmer Umgebung. z.B. in Bädern. Solche Einrichtungen sind bevorzugte Lebensräume für Bakterien, ebenso wie bestimmte Oberflächen in Bereichen mit hohem Publikumsverkehr, so z.B. in öffentlichen Verkehrsmitteln. Krankenhäusern. Telefonzellen, Schulen und insbesondere in öffentlichen Toiletten.

In der Alten- und Krankenpflege erfordern die häufig geminderten Abwehrkräfte der Betroffenen sorgfältige Maßnahmen gegen Infektionen, insbesondere auf Intensivstationen und bei häuslicher Pflege.

Es ist nun eine Aufgabe der Erfindung, Gegenstände verfügbar zu machen, deren bestimmungsgemäße Verwendung eine von Mikroorganismen zumindest möglichst freie Oberfläche erfordert, deren Oberfläche einen niedrigen Reibungskoeffizienten hat und bei Verwendung im menschlichen oder tierischen Organismus keine toxische Wirkung auf deren Zellen ausübt. Eine weitere Aufgabe der Erfindung besteht darin, ein Verfahren zur Herstellung solcher Gegenstände mittels zweckentsprechender Beschichtungsmittel bereitzustellen. Schließlich ist eine Aufgabe der Erfindung, für die erwähnten Gegenstände einer zweckentsprechende Verwendung vorzuschlagen.

Die erste dieser Aufgaben wird gelöst durch einen Gegenstand aus Metall, Keramik oder Kunststoff, der durch eine Mikroorganismen abweisende Beschichtung aus cyanalkylierter Hydroxyalkylzellulose gekennzeichnet ist.

Die zweite Aufgabe wird gelöst durch ein Verfahren, bei dem man den Gegenstand mit cyanethylierter Hydroxyalkylzellulose beschichtet.

Die dritte Aufgabe wird gelöst durch Verwendung des genannten Gegenstandes für Zwecke, bei denen es darauf ankommt, daß die Oberfläche des Gegenstandes möglichst weitgehend frei von Mikroorganismen bleibt.

Die Gegenstände nach der Erfindung sind, wie gesagt, dazu bestimmt, unter Bedingungen verwendet zu werden, bei denen es darauf ankommt, daß die Oberfläche möglichst frei von Mikroorganismen ist. Nach einer anderen Formulierung für diesen Sachverhalt erfordert ihre Verwendung, daß die Oberfläche abweisend für Mikroorganismen ist. Die Bezeichnung "möglichst frei" schließt natürlich "völlig frei" ein. Hierbei handelt es sich um einen idealtypischen Grenzfall. Die Vorteile der Erfindung zeigen sich aber auch in einer im Vergleich zu konventionellen Gegenständen deutlich geringeren Dichte der Besiedelung oder Belegung mit Mikroorganismen.

Die Bezeichnung Mikroorganismen ist für die Zwecke dieser Erfindung weit auszulegen. Es handelt sich um mikroskopisch kleine Zellen oder zellähnliche Gebilde. Im einzelnen seien Bakterien, insbesondere pathogene Bakterien; Hefen und Pilze genannt. Auch die erwähnten zellähnlichen Gebilde des Blutes, wie Thrombocyten, gelten als Mikroorganismen im Sinne der Erfindung.

Die Gegenstände nach der Erfindung eignen sich zur Verwendung auf den verschiedensten Gebieten, auf denen bakterienabweisende Oberflächen erwünscht sind. Sie können dementsprechend z.B. für medizinische oder technische Zwecke einschließlich lebensmitteltechnischer Zwecke sowie auf den Gebieten der Pharmazie, Biologie, Pflege, Hygiene oder Ernährung verwendet werden.

Von den drei genannten Eigenschaften - abweisend für Mikroorganismen, niedriger Reibungskoeffizient und keine Cytotoxizität - ist die erstgenannte die wichtigste, weil es auf sie bei jeder Ausführungsform der Erfindung ankommt. Es gibt jedoch Verwendungen, insbesondere auf nicht-medizinischem Gebiet, bei denen eine der übrigen oder beide übrigen Eigenschaften möglicherweise nicht oder nur von geringer Bedeutung, aber trotzdem natürlich vorhanden sind. Die Gegenstände nach der Erfindung eignen sich insbesondere zur Verwendung auf medizinischem Gebiet, insbesondere für therapeutische oder diagnostische Zwecke, und sind dann beispielsweise Katheter, Stents, Dialyse- oder Katheterschläuche, künstliche Blutgefäße, Herzklappen, Gelenke und Linsen sowie Kontaktlinsen oder chirurgisches Nahtmaterial. Sie eignen sich aber auch zur Verwendung auf allen anderen Gebieten, wenn es auf die genannten Eigenschaften der Gegenstände ankommt. Beispiele hierfür sind die Herstellung von Arzneimitteln unter sterilen Bedingungen, wobei die erfindungsgemäßen Gegenstände Apparate und Apparateteile, Behälter, Rohrleitungen, Verpackungs- und Versandmittel usw. sein können.

Die Gegenstände nach der Erfindung können aus den verschiedensten Werkstoffen bestehen. Beispiele für metallische Werkstoffe sind Eisen, Stahl, insbesondere hochlegierter, rostfreier Stahl, sowie Nickel, Titan, Mangan und Aluminium. Für die Erfindung geeignete keramische Werkstoffe sind die aus anorganischen, überwiegend nichtmetallischen Elementen aufgebauten, gegebenenfalls teilweise kristallinen Materialien, die als Gläser, Glaskeramiken oder Oxidkeramiken bekannt sind. Die keramischen Werkstoffe können auch statt der oder neben den oxidische(n) Anteilen Nitride, Boride, Carbide und/oder Silicide enthalten. Von ähnlicher Vielfalt sind die für die Erfindung geeigneten Werkstoffe auf Basis von Kunststoffen. Die letzteren können Polymerisate, Polykondensate oder Polyadditionsverbindungen sein. Als Beispiele seien Polyolefine, wie Polyethylen oder Polypropylen; Copolymere des Ethylens und Propylens miteinander und/oder mit anderen olefinisch ungesättigten Monomeren, wie Buten-1, Vinylacetat und Acrylnitril; Polyester, wie Polyethylen- und Polybutylenterephthalat; Polycarbonate; Polyamide, wie Polycaprolactam und Polylaurinlactam; Polyalkylenfluoride, wie Polyvinylidenfluorid und Polytetrafluorethylen; Polyurethane usw., Natürlich fallen auch Gegenstände in den Bereich der Erfindung, die aus Kombinationen verschiedener Werkstoffe bestehen, z.B. aus verschiedenen Metallen oder aus einem Metall und einem Kunststoff. Bei medizinischer oder anderer Verwendung im Zusammenhang mit lebenden Makroorganismen ist bei der Auswahl der Werkstoffe die Biokompatibilität zu beachten.

Cyanalkylierte Hydroxyalkylzellulose ist ein bekanntes Material, dessen Herstellung und Eigenschaften von JW Mays in Macromolecules **21**, 3179-83 1988 beschrieben wurden. In der DE A1-40 40 363 wird die Verwendung von cyanethylierter Hydroxypropylzellulose für die Beschichtung von porösen und kompakten Substraten beschrieben. Im Beispiel 2 wird eine Polyethylenfolie beschichtet, in den Beispiel 7 bis 9 mikroporöse Membranen aus Polypropylen. Die beschichteten Membranen wurden auf Durchlässigkeit und Selektivität für die Trennung von Gasgemischen geprüft.

Bei der Cyanalkylierung geht man von Hydroxyalkylzellulosen aus, die in der Regel ein mittleres Molekulargewicht von 50.000 bis 2.000.000 haben, z.B. von Hydroxyethyl- oder -propylzellulose, und im Handel erhältlich sind. Das Produkt wird in einem inerten Lösemittel, wie tert.-Butanol, gelöst und in Gegenwart eines Katalysators mit der berechneten Menge alpha,beta-ungesättigtem Nitril, vorteilhaft Acrylnitril versetzt. Man wendet zweckmäßig 0.1 bis 3 Mol Acrylnitril je Mol Glukosebaustein oder einen Überschuß an, der bis zum 5-fachen der äquivalenten Menge, bezogen auf 3 reaktive OH-Gruppen des Glukosebausteins, beträgt. Geeignete Katalysatoren sind z.B. quaternäre Ammoniumhydroxide, wie Benzyltrimethylammoniumhydroxid.

Die Gegenstände nach der Erfindung werden hergestellt, indem die zweckentsprechend geformten Substrate aus den jeweilige Materialien in bekannter Weise, z.B. durch Tauchen, Spritzen oder Streichen, mit Lösungen der cyanalkylierten Hydroxyalkylzellulose beschichtet. Die Mikroorganismen abweisenden Eigenschaften der Gegenstände nach der Erfindung sind besonders ausgeprägt, wenn die Oberfläche des Films eben und porenfrei ist. Eine ebene und porenfreie Oberfläche des Films läßt sich am leichtesten erzeugen, wenn die Substrate ihrerseits eben und porenfrei sind. Solche Substrate werden daher bevorzugt, weil die auf ihnen erzeugten Filme ebenfalls eben und porenfrei sind. Ein bewährtes, leicht flüchtiges Lösemittel ist Aceton. Nach dem Verdampfen des Lösemittels und Trocknen. z.B. an der Luft und gegebenenfalls bei mäßig erhöhter Temperatur, haftet der Film aus der cyanalkylierten Hydroxyalkylzellulose bemerkenswert fest auf den verschiedensten Substraten.

Der Film ist hydrophob und nicht wasserlöslich, quillt aber unter Wasseraufnahme, je nach dem Cyanalkylierungsgrad, mehr oder weniger stark auf. Durch Plasmabehandlung oder Corona-Entladung bzw. durch Bestrahlen mit energiereichen Strahlen, d.h. solchen Strahlen, die energiereicher als sichtbares Licht sind, können die Filme, möglicherweise unter Vernetzung, so modifiziert werden, daß sie in Aceton praktisch unlöslich sind und in Wasser nicht mehr nennenswert quellen. Zu den energiereichen Strahlen zählen UV-, Röntgen- und Elektronenstrahlen. Bevorzugt wird die Modifizierung mit UV-Strahlen. Die modifizierten Filme zeigen eine noch bessere Haftung, besonders auf glatten, porenfreien Substraten. Die Mikroorganismen abweisenden Eigenschaften, der erwünschte niedrige Reibungskoeffizient sowie die günstigen cytotoxischen Eigenschaften der Beschichtungen werden durch die Modifizierung nicht beeinträchtigt.

Die folgenden Beispiele sollen die Erfindung erläutern, nicht jedoch- ihren Anwendungsbereich begrenzen. Alle Prozentangaben beziehen sich auf das Gewicht, soweit nicht anders angegeben.

### Beispiel 1 - Herstellung von cyanethylierter Hydroxypropylzellulose

Einer 2.5%-igen Lösung von Hydroxypropylzellulose (Molgewicht ca. 1.000.000. Aldrich Chemie GmbH & Co. KG. Steinheim) in tert.-Butanol werden 0,03% Benzyltrimethylammoniumhydroxid als Katalysator zugesetzt. Dann wird ein 5-facher molarer Überschuß Acrylnitril pro Glukosebaustein mit 3 Hydroxylgruppen zugetropft. Man erwärmt das Gemisch 2h auf 40°C und engt es anschließend im Vakuum ein. Die hochviskose Lösung wird mit Aceton verdünnt und in Essigsäure enthaltendes Wasser, pH 3,5. eingetragen, wodurch die cyanethylierte Hydroxypropylzellulose ausgefällt wird. Die Ausbeute beträgt nach erneuter Umfällung 60% d.Th., der Stickstoffgehalt des getrockneten Produkts nach Elementaranalyse 3,7%.

### Beispiel 2 - Benetzbarkeit der Beschichtungen

Verschiedene Kunststoffolien wurden auf eine Präpariernadel aufgespießt und in eine 1%-ige acetonische Lösung von cyanethylierter Hydroxypropylzellulose (AHC) nach Beispiel 1 getaucht. Überschüssige Lösung wird abgeschüttelt und die Folie an der Luft getrocknet. Ein Tropfen Wasser wird auf den Film aufgebracht und mit einer Videokamera aufgenommen. Das Bild wird digitalisiert und der Kontaktwinkel bestimmt, den der Tropfen mit der Oberfläche bildet.

**Tabelle 1**

| Benetzungswinkel (Grad) von Wasser auf unbeschichteten und mit cyanethylierter Hydroxypropylzellulose beschichteten Kunststoffolien | | | |
|---|---|---|---|
| Folie | unbeschichtet | AHC 300/5 | AHC 5Mio/5 |
| Polyamid 12 | 82,5 | 97 | 117 |
| Polyethylen | 96 | 87 | 113 |
| Polypropylen | 99 | 90 | 114 |
| Ethylen-Vinylacetat | 86 | 76 | 115 |
| Polyurethan | 89 | 87 | 121 |
| Polytetrafluorethylen | 106 | 87 | 104 |

Die Werte sind das Mittel aus 5 Einzelmessungen.

Die Beschichtung bewirkt im wesentlichen keine Hydrophilierung der Kunststoffoberflächen. Das Produkt AHC 300/5 (Hydroxypropylzellulose vom Molgewicht 300.000 mit 5mol Acrylnitril pro mol Glukosebaustein) ergibt überwiegend eine geringe Hydrophilierung der Trägerfolie, das Produkt aus der Umsetzung der Hydroxypropylzellulose vom Molgewicht 1.000.000 eine Hydrophobierung.

### Beispiel 3 - Bakterielle Adhäsion

Runde Folienstücke von 1,6cm Durchmesser werden wie in Beispiel 2 mit AHC beschichtet. Dann werden die beschichteten Folien und zum Vergleich auch unbeschichtete Folien 4h lang in einer Suspension von lebenden Bakterien des Stammes Klebsiella pneumoniae in phosphatgepufferter Saline (PBS) geschüttelt und anschließend zweimal mit PBS gewaschen. Aus den an der Kunststoffolie anhängenden Bakterien wird mit siedendem TRIS/EDTA-Puffer das in den Zellen vorhandene Adenosintriphosphat (ATP) extrahiert und luminometrisch mit einem Reagenziensatz von Boehringer Mannheim bestimmt. Die gemessenen Lichteinheiten (RLU) sind ein Maß für die Anzahl der an einer Folie haftenden Bakterien. In der Tabelle 2 ist die Veränderung der bakteriellen Adhäsion nach Beschichtung verschiedener Kunststoffe mit AHC wiedergegeben.

**Tabelle 2**

| Veränderung der bakteriellen Adhäsion auf verschiedenen Kunststoffen durch Beschichtung mit AHC (ausgedrückt in RLU x 10⁻³) | | | |
|---|---|---|---|
| Folie | unbeschichtet | AHC 300/5 | AHC 5Mio/5 |
| Polyamid 12 | 64,5 | 15,7 | 18,1 |
| Polyethylen | 44,2 | 6,6 | 33,5 |
| Polypropylen | 50,5 | 30,1 | 10,0 |
| Ethylen-Vinylacetat | 26,2 | 14,7 | 5,3 |
| Polyurethan | 32,3 | 20,6 | 23,7 |
| Polytetrafluorethylen | 126,0 | 37,8 | 24,4 |

Bei allen beschichteten Kunststoffen wurde eine deutlich verminderte bakterielle Adhäsion festgestellt.

### Beispiel 4 - Adhäsion von Thrombocyten

Runde Folienstücke von 0,8cm Durchmesser werden wie in Beispiel 2 mit AHC beschichtet. Die beschichteten Folienstücke und zum Vergleich auch unbeschichtete Folienstücke werden 30min lang in Citrat-haltigem Kaninchenblut geschüttelt und anschließend zweimal mit PBS gewaschen. Die relative Anzahl der auf der Oberfläche adhärierenden Thrombocyten wird durch ATP-Extraktion aus den durch den Waschvorgang nicht entfernten Thrombocyten mit siedendem TRIS/EDTA-Puffer bestimmt. Die aus den Thrombocyten extrahierte ATP-Menge wird, wie in Beispiel 3 beschrieben, luminometrisch bestimmt. In Tabelle 3 ist die relative Anzahl der adhärierenden Thrombocyten mittels der aus ihnen extrahierten ATP-Menge, ausgedrückt in RLU, wiedergegeben.

**Tabelle 3**

| Veränderung der Thrombocyten-Adhäsion auf verschiedenen Kunststoffen durch Beschichtung mit AHC (RLUx10⁻³) | | | |
|---|---|---|---|
| Folie | unbeschichtet | AHC 300/5 | AHC 5Mio/5 |
| Polyamid 12 | 424,3 | 6,0 | 26,1 |
| Polyethylen | 129,2 | 9,5 | 2,1 |
| Tabelle 3 (Fortsetzung) Polypropylen | 155,1 | 3,7 | 5,1 |
| Ethylen-Vinylacetat | 159,0 | 65,2 | 5,9 |
| Polyurethan | 127,1 | 2,4 | 3,6 |
| Polytetrafluorethylen | 170,8 | 5,6 | 9,3 |

Es wurde eine Verminderung der Thrombocyten-Adhäsion um bis zu etwa 98% festgestellt. Eine Hämolyse des Citrat-Blutes durch die beschichteten oder unbeschichteten Kunststoffe wurde nicht beobachtet.

### Beispiel 5 - Cytotoxicität

Zur Bestimmung der Cytotoxizität der beschichteten und der unbeschichteten Folien wurden die Folien durch Eintauchen in 70-volumenprozentiges Ethanol entkeimt und nach dem Trocknen in eine Nährlösung von 5g Proteose-Pepton, 4g Pepton aus Casein, 1g Fleischextrakt und 200mg K₂HPO₄ pro Liter gelegt. Die Nährlösung wurde mit dem Ciliaten Tetrahymena pyriformis geimpft. Die Hemmung des Wachstums dieses Ciliaten kann als Indikator für die cytotoxische Wirkung gewertet werden (DIN 13273, Teil 5, November 1986). Dabei ist es wiederum möglich, die Zahl der lebenden Zellen über die Menge des extrahierbaren ATP zu bestimmen. Das extrahierte ATP wird wie in Beispiel 3 luminometrisch bestimmt. Eine cytotoxische Wirkung der AHC-Beschichtung gegenüber unbeschichteten Folien war nicht festzustellen. Dagegen hatte eine Kontrollfolie aus Weich-PVC mit dem Konservierungsstoff Oxybisphenoxyarsin eine ausgeprägte cytotoxische Wirkung.

### Beispiel 6 - Reibungskoeffizient

Ein Katheterschlauch aus Polyurethan (Pellethane^{(R)}2363-90A) wurde für 2sec in eine 2 %-ige acetonische Lösung von cyanalkylierter Hydroxypropylzellulose getaucht, über Nacht bei Raumtemperatur und 1 h bei 40°C getrocknet. Zur Messung des Reibungskoeffizienten wurden die Schläuche mit destilliertem Wasser befeuchtet und dann durch ein passende Öse gezogen. Der gemessene Reibungskoeffizient ist in Tabelle 4 wiedergegeben.

**Tabelle 4**

| Beschichtung | Reibungskoeffizient |
|---|---|
| unbeschichtet | 0,66 |
| AHC 1 Mio/5 | 0,34 |
| AHC 300/5 | 0,51 |

Der Reibungskoeffizient ist durch die Beschichtung mit AHC reduziert worden.

### Beispiel 7 - Löslichkeit nach Modifizierung durch UV-Strahlen

Die gemäß Beispiel 2 behandelten, porenfreien Folien wurden in einer Anlage zur UV-Behandlung (UV-Excimer, Heraeus, Type Exivac VAC; 1,7 kW) zweimal auf jeder Seite jeweils 1 min im Abstand von 4 cm im Vakuum bei 1 mbar mit 172 nm bestrahlt. Die Bakterien und Thrombozyten abweisende Eigenschaft der Beschichtung wurde durch die Bestrahlung nicht verändert.

Die Löslichkeit der bestrahlten und der unbestrahlten Beschichtung in Aceton wurde geprüft, indem ein 5x7 cm großes Folienstück von 220mg 10 min mit 10ml Aceton geschüttelt, an der Luft getrocknet und gewogen wurde

**Tabelle 5**

| Extraktion von Feststoff mit Aceton aus beschichteten und unbeschichteten Polypropylen (PP)-Folien | | |
|---|---|---|
| Folie | UV-Bestrahlung | mg extrahierter Feststoff |
| PP | - | 0,2 |
| PP | + | 0,4 |
| PP + AHC 300/5 | - | 7,7 |
| PP + AHC 300/5 | + | 0,6 |
| PP + AHC 1Mio/5 | - | 6,9 |
| PP + AHC 1Mio/5 | + | 0,4 |

Die AHC-Beschichtungen sind durch die UV-Bestrahlung praktisch unlöslich geworden.

## Patentansprüche

1. Gegenstand aus Metall. Keramik oder Kunststoff, dessen Verwendung eine von Mikroorganismen möglichst freie Oberfläche erfordert, gekennzeichnet durch eine Mikroorganismen abweisende Beschichtung aus cyanethylierter Hydroxyalkylzellulose.

2. Gegenstand nach Anspruch 1, der für medizinische Zwecke bestimmt ist.

3. Gegenstand nach Anspruch 1 oder 2, gekennzeichnet durch eine porenfreie Oberfläche der Mikroorganismen abweisenden Beschichtung.

4. Gegenstand nach einem der Ansprüche 1 bis 3, gekennzeichnet durch eine porenfreie Oberfläche des unbeschichteten Gegenstands.

5. Gegenstand nach einem der Ansprüche 1 bis 4, gekennzeichnet durch eine mittels Plasmabehandlung, Corona-Entladung oder Bestrahlen mit energiereicher Strahlung modifizierte Mikroorganismen abweisende Beschichtung.

6. Gegenstand nach einem der Ansprüche 1 bis 5, dadurch gekennzeichnet, daß er ein Katheter oder ein Stent ist.

7. Verfahren zur Herstellung einer Mikroorganismen abweisenden Beschichtung auf einem Gegenstand aus Metall, Keramik oder Kunststoff, dadurch gekennzeichnet, daß der Gegenstand mit cyanethylierter Hydroxyalkylzellulose beschichtet wird.

8. Verfahren nach Anspruch 7, dadurch gekennzeichnet, daß die Mikroorganismen abweisende Beschichtung porenfrei ist.

9. Verfahren nach Anspruch 7 oder 8, dadurch gekennzeichnet, daß der unbeschichtete Gegenstand porenfrei ist.

10. Verwendung des Gegenstands nach einem der Ansprüche 1 bis 6 für Zwecke, bei denen es darauf ankommt, daß die Oberfläche möglichst weitgehend frei von Mikroorganismen ist.

11. Verwendung nach Anspruch 10, dadurch gekennzeichnet, daß der Gegenstand für medizinische Zwecke verwendet wird.

12. Verwendung nach Anspruch 10, dadurch gekennzeichnet, daß der Gegenstand für medizinische oder technische Zwecke einschließlich lebensmitteltechnischer Zwecke oder auf den Gebieten der Pharmazie, Biologie, Pflege, Hygiene oder Ernährung verwendet wird.
